# EUROPEAN PATENT APPLICATION

(11) **EP 1 762 267 A1**
(43) Date of publication of application: **14.03.2007**
(21) Application number: 05739158.3
(22) Date of filing: 10.05.2005
(51) Int. Cl.: A61N 1/10, A61N 1/40, A61D 1/10

(54) **NONPHARMACOLOGICAL ELECTRIC FIELD METHOD AND APPARATUS FOR TREATING AND AMELIORATING RHEUMATISM AND PAIN**

(30) Priority: 11.05.2004 JP 2004141543
(71) Applicant: Hakuju Institute for Health Science Co., Ltd, Tokyo 151-0063 (JP); School Juridical Person Nihon University, Tokyo 102-8275 (JP)
(72) Inventor: HARA, Akikuni, HAKUJU I. FOR H. SCIENCE CO., LTD., Tokyo 1510063 (JP); YUKAWA, Masayoshi, c/o NIHON UNIVERSITY, Tokyo 1028275 (JP); KUWABARA, Masato, c/o NIHON UNIVERSITY, Tokyo 1028275 (JP); HARAKAWA, Shinji, HAKUJU I. FOR H. SCIENCE CO LTD, Tokyo 1510063 (JP); HORI, Takuya, HAKUJU I. FOR HEALTH SCIENCE CO. LTD, Tokyo 1510063 (JP); DOGE, Fuyuki, HAKUJU I. FOR HEALTH SCIENCE CO. LTD, Tokyo 1510063 (JP)
(74) Representative: Senior, Janet
(86) International application number: PCT/JP2005/008516
(87) International publication number: WO 2005/107853

(57) **Abstract**

The present invention relates to a method of treating or improving rheumatism and pain caused by rheumatism, tumor, and spinal damage of mammalian animals by a non-pharmacological method. Extremely low AC electric field with DC electric field overlapped is exposed to a whole or a part of mammalian animals with electrodes in a non-contact state. In the exposure of the above mentioned electric field, said electric field is exposed for a certain period of time a day, repeating this everyday or once a week. Further, as required, frequency of the electric field, voltage to be applied, exposure time, and the like are controlled.

## Description

The description of this application claims benefit of priority based on Japanese Patent Application No.2004-141543, the entire same contents of which are incorporated by reference herein.

### BACKGROUND OF THE INVENTION

### Field of the Invention

The present invention relates to methods for treating or improving rheumatism and pain and its apparatus therefor.

In more detail, it relates to non-pharmacological methods for treating or improving rheumatism and pain caused by the rheumatism, tumor, spinal damage, and the like by exposure of electric fields, and its apparatus therefor.

### Description of the Prior Art

Rheumatism is disease causing pain, swelling, inflammation to joints of a whole body including limbs, and as the state of the disease develops, it causes deformation or dysfunction of the joints.

For treating rheumatism, various kinds of medications have been administered. Steroidal therapeutic medication, one of the typical medications, has a drawback of strong side effects. For example, although a steroidal therapeutic medication with fewer side effects as disclosed in a patent document 1 has been developed, all the side effects cannot be eliminated. Even when non steroidal therapeutic medications are administered, side effects might be caused to digestive systems and the like, and this has been the problem which remains unsolved.
Patent Document 1: Japan Unexamined laid-open patent publication 2002-539167

### SUMMARY OF THE INVENTION

As the state of the disease of rheumatism further develops, an operation for removing the synovial membrane of the inflamed joint is required, and when it further develops, an operation of replacing the deformed joint with an artificial one is sometimes performed. Both operations are heavy burden for patients, especially for aged patients.

Not only human beings but also other mammalian animals in particular, pet animals such as dogs, cats, and the like can develop rheumatism. Therefore, owners for pet animals have also longed for effective methods for treating rheumatism.

In addition, not only rheumatism but also chronic pain caused by tumor, spinal damage, and the like are intolerable for patients and thus, effective methods for treating such disease have also been longed for.

The object of the present invention is to provide methods for treating rheumatism and pain caused by rheumatism, tumor, spinal damage, and the like by non-pharmacological methods, and its apparatus.

The present invention achieves the above mentioned object by which rheumatism of mammalian animals is treated or improved non-pharmacologically, and in which electric fields are exposed to whole or a part of mammalian animals with electrodes in a non-contact state with said mammalian animals.

Although detailed mechanisms have not been found out, it is considered that by exposing electric fields, biosynthetic pathways of beta-endorphin and decomposition pathways of ACTH in hypophysis, particularly in intermediate lobe are activated directly or indirectly thereby treating and improving symptoms of rheumatism.

In addition, in treating rheumatism, affected areas of the rheumatism are improved organically by exposing electric fields at least to the affected areas.

It is preferable that the electric fields are the extremely low frequency AC electric fields and it is more preferable that the AC electric fields are overlapped with DC electric fields.

It is preferable that regarding the exposure of the electric fields, the electric fields are exposed only for a certain period of time in a day, repeating it everyday or with certain intervals, and it is more preferable that the frequency of the exposure is more than once a week. By this, the symptom is gradually improved.

Although available to all the species as long as they are mammalian animals, pet animals such as dogs, cats, and the like are particularly available. Further, the method can treat and improve human beings.

Also, the present invention relates to a treating and improving apparatus which treats or improves rheumatism of mammalian animals, wherein said apparatus is provided with a pair of electrodes which can expose electric fields to a whole body or a part including rheumatism-affected part of mammalian animals and a power source which applies voltage to said pair of electrodes.

Another embodiment of the present invention includes an apparatus by which the affected areas can be improved organically by exposing the electric fields at least to the affected areas of rheumatism. It relates to the apparatus in which the above mentioned synthetic paths and decomposition paths of hormones are activated by exposing the electric fields at least to the diseased area thereby treating and improving the symptoms of rheumatism.

It is preferable that the electric fields generated by the apparatus of the present invention are the extremely low frequency AC electric fields and it is more preferable that the AC electric fields are overlapped with DC electric fields. It is preferable that the present apparatus of the present invention is further provided with a power controller by which at least one among applied voltage, frequency, and exposure time is controllable depending on the size or symptom of mammalian animals to be treated.

The apparatus related to the present invention is the apparatus effective for treating or improving rheumatism and it is particularly effective for pet animals such as dogs, cats, and the like, and human beings although species of mammalian animals are not specifically limited.

Another embodiment of the present invention includes an apparatus for treating or improving rheumatism of mammalian animals and pain caused by rheumatism, tumor, spinal damage, and the like, thereby preparing the apparatus for treating or improving provided with a pair of electrodes in which electric fields can be exposed to a whole body or a part thereof including affected area of said mammalian animals and a power source applying voltage to said pair of electrodes.

It is preferable that the electric fields generated by the present apparatus are the extremely low frequency AC electric fields and it is more preferable that the AC electric fields are overlapped with DC electric fields.

It is preferable that the present apparatus of the present invention is further provided with a power controller by which at least one among applied voltage, frequency, and exposure time is controllable depending on the size or symptom of mammalian animals to be treated.

The apparatus related to the present invention is the apparatus effective for treating or improving rheumatism and pain caused by rheumatism, tumor, and spinal damage and it is particularly effective for pet animals such as dogs, cats, and the like, and human beings although species of mammalian animals are not specifically limited.

According to the method and the apparatus of the present invention, other than rheumatism of mammalian animals, in particular, that of pet animals such as dogs, cats, and the like, and human beings, pain caused by rheumatism, tumor, and spinal damage can be treated or improved. That is, it is considered that by exposing electric fields, synthetic paths of beta-endorphin and decomposition paths of ACTH are indirectly or directly activated in hypophysis, in particular, in intermediate lobe, thereby treating or improving symptoms by pain.

### BRIEF DESCRIPTION OF THE DRAWINGS

Fig. 1 is a view showing the treating and improving apparatus related to the present invention. A shows a high voltage generating unit and B shows the electric field exposure cage.
Fig.2 is a view which explains a measuring circuit for electric field strength. A shows a measuring path of the electric field strength, and B shows principle of a Pockels effect, respectively.
Fig.3 is a view which shows the result of electric field strength measurement. A shows 66 measurement points of a bottom surface portion of a cage, and B shows distribution of electric field strength, respectively.
Fig. 4 is a view which shows changes in ACTH values of normal animals before and after electric field exposure.
Fig.5 is a view which shows changes in beta-endorphin values of normal animals before and after electric field exposure.
Fig. 6 is a view which shows changes inACTH values of subject animals with tumor before and after electric field exposure.
Fig.7 is a view which shows changes in beta-endorphin values of subject animals with tumor before and after electric field exposure.
Fig. 8 is a view which shows changes inACTH values of subject animals with spinal damage before and after electric field exposure.
Fig.9 is a view which shows changes in beta-endorphin values of subject animals with spinal damage before and after electric field exposure.
Fig. 10 is a view showing a subject animal in the cage with chronic articular rheumatism.
Fig. 11 is a view which shows changes in CRP values of subject animals with chronic articular rheumatism before and after electric field exposure.
Fig. 12 is a view which shows changes in CRP values of subject animals with chronic articular rheumatism in continuous treatment.
Fig.13 is a view which shows changes in beta-endorphin values of subject animals with chronic articular rheumatism in continuous treatment.
Fig.14 is a view which shows an MRI image of a rheumatism-affected area before and after electric field exposure.
Fig.15 is a view which shows an electric field exposure apparatus for human beings related to the present invention.
Fig. 16 is a view which shows changes in CRP values of subject animals with chronic articular rheumatism after electric field exposure.
Fig. 17 is a view which shows changes in CRP values of subject animals with chronic articular rheumatism before electric field exposure.

### DETAILED DESCRIPTION OF THE PREFERRED EMBODIMENT

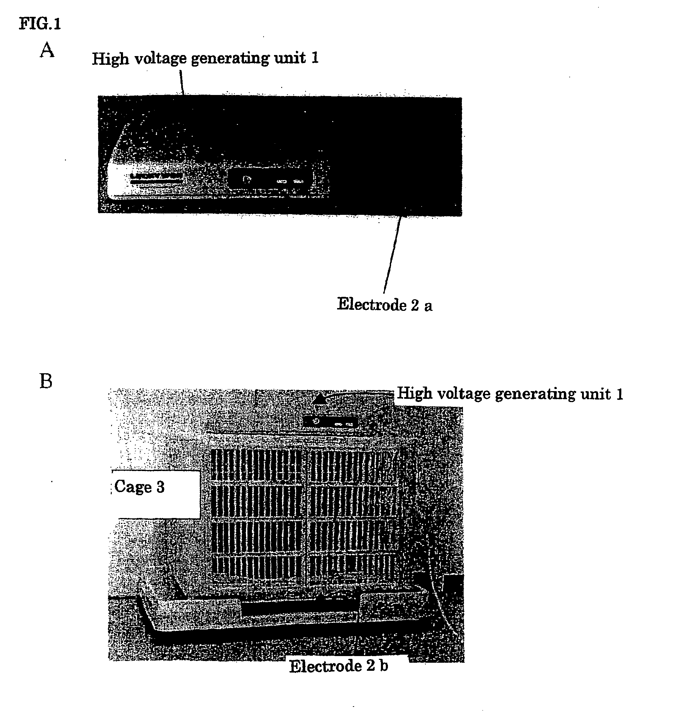

Hereinafter, referring to the drawings, embodiments of the present invention are explained. Fig.1 shows one example of a treating and improving apparatus related to the present invention. Fig.1 A shows a high voltage generating unit and at the bottom surface of the high voltage generating unit 1, one of a pair of electrodes 2 a is provided. Fig.1 B shows an electric field exposure cage 3 and a high voltage generating unit 1 and the other of a pair of electrodes 2 b is provided at the bottom surface of an electric field exposure cage 3.

A mammalian animal suffering from pain is placed in an electric field exposure cage 3. When high voltage generated from a high voltage generating unit 1 was applied to a pair of electrodes 2 a and 2 b, electric fields are generated in an electric field exposure cage 3 and the electric field is exposed to the mammalian animal. Further, the electric field is exposed to the mammalian animal in the cage 3 without contacting any of electrode 2 a or 2 b.

Although the voltage applied to the electrodes 2 a and 2 b may be either of AC or DC, it is preferable that the electric fields are the extremely low frequency AC electric fields and it is more preferable that the AC electric fields are overlapped with DC electric fields. Further, the high voltage generating unit 1 includes a controller (not illustrated) which controls frequency, applied voltage, exposure time, and the like.

In addition, by exposing the electric fields for a certain period of time a day and repeating it every day, the treating and improving effect is gradually shown.

Although the relationship between electric field exposure and the effect of treating pain is not unraveled, it is assumed that, as mentioned above, synthesis of beta-endorphin and decomposition of ACTH are promoted, thereby showing the effect of treating (improving) pain.

In order to acknowledge the treating and improving effect of pain by the present invention, the treatment experiments were conducted on dogs as subject animals.

### EXAMPLE 1

### (Treating and improving apparatus)

A treating and improving apparatus is composed of two major parts, namely a high voltage generating unit 1 (Fig.1 A) and an electric field exposure cage 3 (Fig.1B). The high voltage generating unit 1 measures 500 mm in width, 360 mm in depth, and 92 mm in height, and has frequency of 50 Hz, and maximum output voltage of 2000 V (overlapped with DC 800 V). The electric field exposure cage 3 is a commercially available one which measures 758 mm in width, 583 mm in depth, and 532 mm in height. One electrode 2a is attached to a bottom portion of the high voltage generating unit 1, and the high voltage generating unit 1 is attached over the cage 3. Further, another electrode 2b (540 mm in width, 420 mm in depth, and 3 mm in thickness) is attached under the cage 3. An electric signal is boosted and converged in the high voltage generating unit 1, and as a result, strong electric fields are generated between electrodes 2a and 2b.

### (Distribution of electric fields)

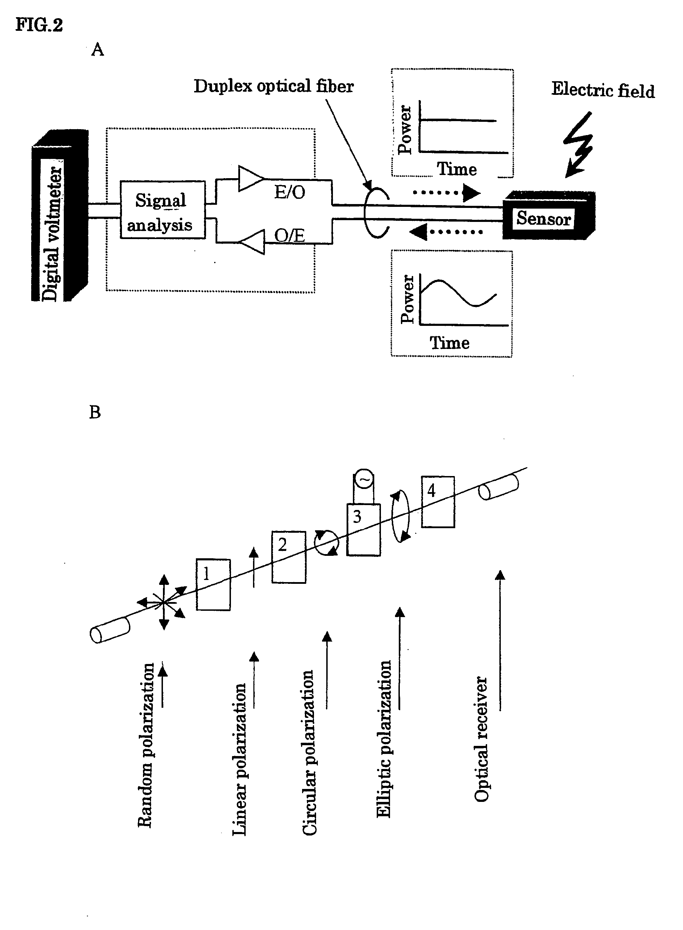

In order to measure distribution in an electric field exposure cage 3, the electric field strength of a bottom space of the cage 3 was measured. Fig.2 A shows a measuring device which is composed of an electro optic voltage sensor to which both a voltmeter which analyses electric field strength by a Pockels effect and Bi₁₂SiO₂₀(BSO) optical fiber (manufactured by Sumitomo Electric Industries, Ltd., Osaka, Japan) are attached. The principle of the voltage sensor is shown in Fig.2 B. In Fig.2 B, p3 is a monocrystal of BSO. When light which accompanies polarized light falls on a BSO crystal exposed by electric fields, the polarized light changes. Since polarized light is converged to light strength by an analyzer, the strength of the electric fields can be measured.

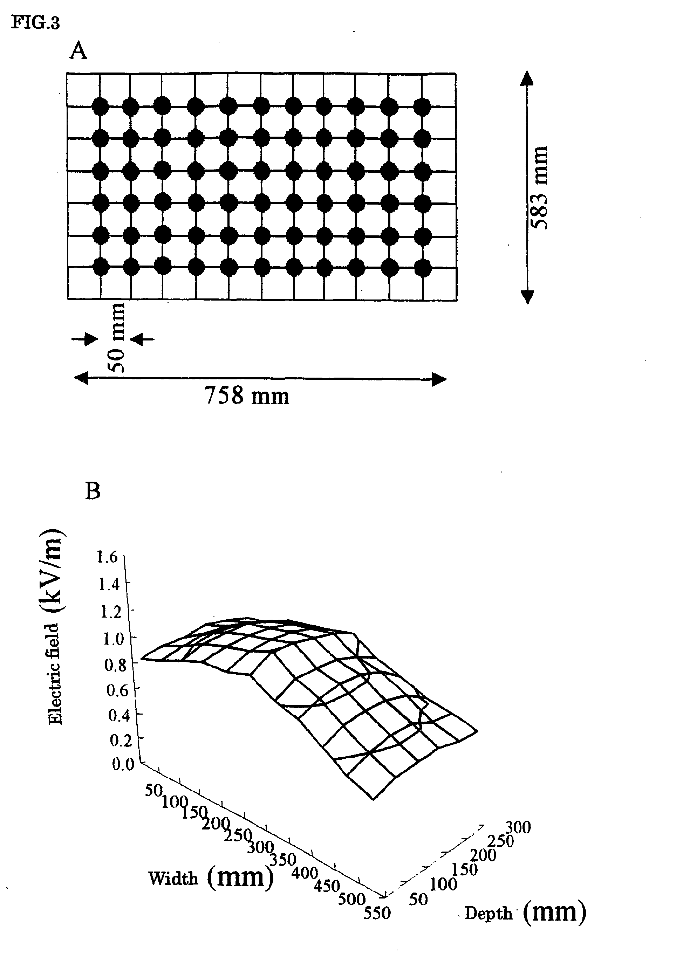

The electric field strength of 66 points at the bottom part of the cage 3 shown in Fig. 3A was measured. This measurement was conducted under the room temperature of 20 ± 1°C and the moisture of 40 ± 2%. A waveform of this research is a 50Hz sine wave overlapped with a DC-component. Fig. 3 B shows distribution of the electric field strength with regard only to an AC-component of the bottom part of exposure cage 3 when maximum generating voltage is applied. The electric field strength of the DC-component is 40 % of the recorded waveform and further, it is considered to be similar to the waveform of the AC-component.

From the result of Fig.3 B, it is found that even when the subject animal moves in the cage 3, the electric fields can fully be exposed to it.

### (Subject animals)

Five male beagle dogs were obtained from Charles Rivers Laboratories as normal animals and further, total of 10 dogs composed of those with tumor (n=5) and those with spinal damage (n=5) were selected from foreign animals brought to Nihon University animal hospital as subject animals.

### (Experimental schedule)

Experiments were conducted at Nihon University animal hospital. Electric fields were exposed to subject animals (dogs) separately, 2 hours a day and over one week. One electric field exposure is defined as one session, and in every session, each subject animal was transferred from the usual cage to the electric field exposure cage 3 and the electric fields were exposed to the animals. In order to avoid imbalanced data caused by change in hormone secretion by circadian rhythm of subject animals, all the experiments were conducted in the time zone from 14:00 to 18:00.

All the animals during the experiments were kept without any other clinical treatment. All the experiments using subject animals were conducted under informed consent.

### (Plasma parameter)

ACTH (adrenocorticotropic hormone) induced by stress and beta-endorphin having morphine-like tranquilizing effect were measured.

Before and after the electric field exposure, from the vessel of forelimb of subject animals, blood samples were collected by injecting heparin. Further, 500 g of the blood samples were centrifugalized for 15 minutes at a temperature of 4°C thereby preparing plasma samples. Plasma samples were preserved at a temperature of -70°C until ACTH and beta-endorphin were measured. ACTH values in the plasma were measured by ACTH radioimmunoassay kit (ACTH IRMA, manufactured by Mitsubishi Chemical Corporation, Tokyo, Japan) and a gamma counter (Auto-Gamma 5530 Gamma Counting System, manufactured by Packard Instrument Corporation). Beta-endorphin values in the plasma were measured by a commercially available kit (manufactured by Mitsubishi Chemical Corporation). These measurements of hormones were conducted at Institute for Health Sciences (Kanagawa, Japan) which is an outside organization.

### (Statistical analysis)

Experimental results are shown by mean ± standard error (S.E.) of mean. Statistical significance of difference between two groups and daily changes were calculated by analysis of variance. The difference between two groups were calculated by a student's test or analysis of variance and the significance was defined to be P< 0.05. All the computations for the statistical analysis were carried out by using MS-EXCEL® Japanese Edition (Microsoft Office software: Ver.9.0.1, Microsoft Japan Inc).

The experiments were conducted based on the guideline on "The Care and Use of Research Animals" published by Panapharm Laboratories.

### (Experimental result)

### Normal subject animals

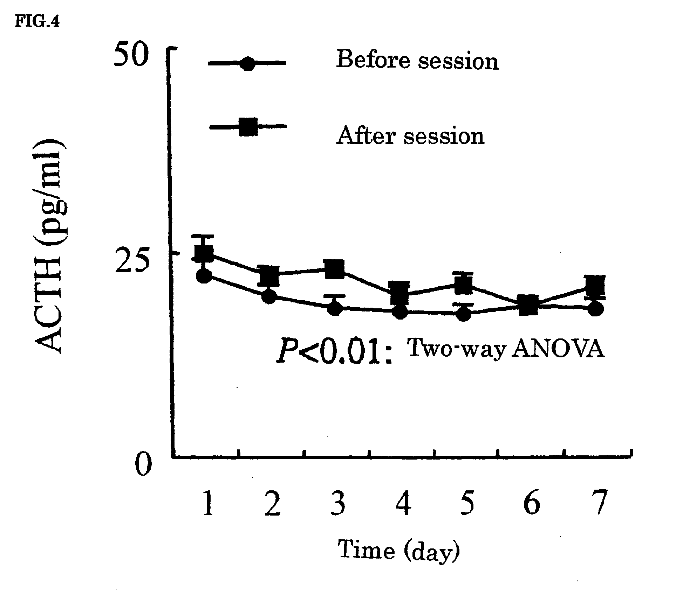

As shown in Fig.4, the ACTH values of the plasma of normal subject animals varied from 17.4±1.05pg/ml to 22.04±2.08pg/ml by mean ± s.e. and a series of data changed daily during seven day experimental term. TheACTH values after each session varied from 18.3±1.18pg/ml to 24.8±2.1pg/ml, showing slight uprise compared with the ACTH values before each session (P<0.01: two-way ANOVA).

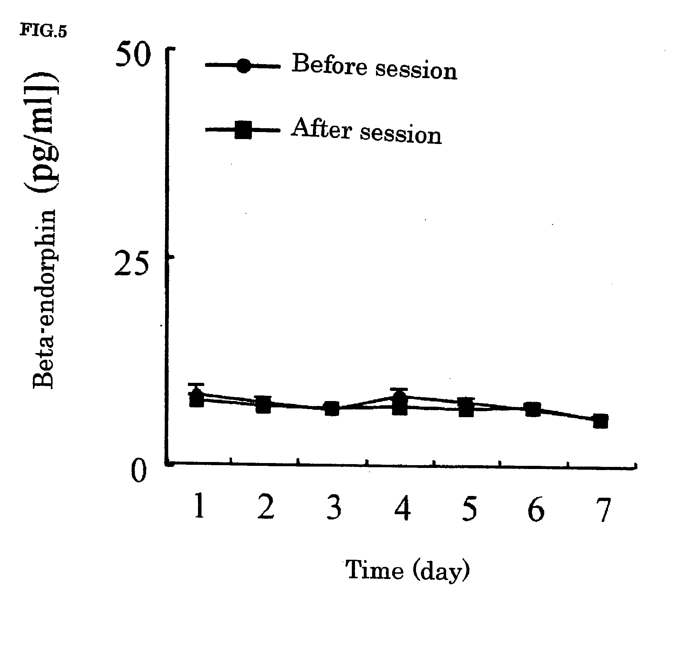

On the other hand, as shown in Fig.5, the beta-endorphin values showed no change at all before and after each session for seven days.

### Subject animals with tumor

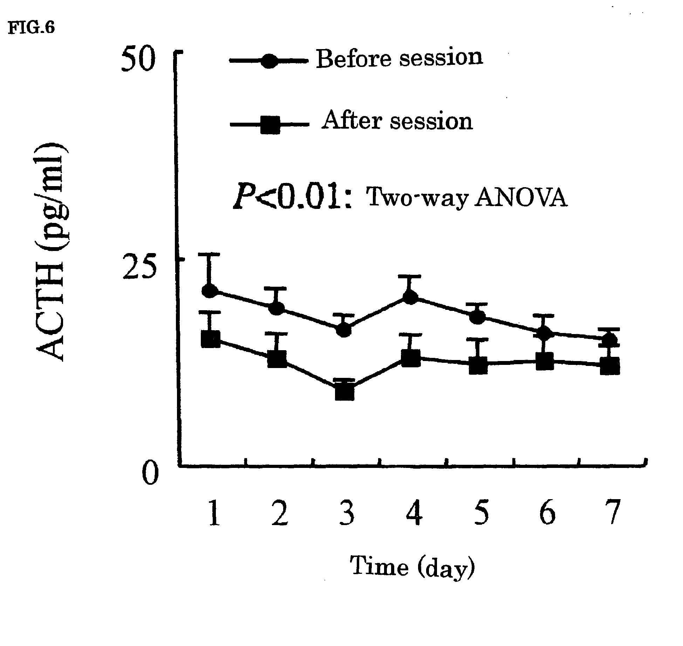

As shown in Fig. 6, although the ACTH values slightly went down on the 3rd day in subject animals with tumor, the ACTH values in the plasma before and after the session saw no remarkable changes in the total of 7 days (P>0.05: one-way ANOVA). The ACTH values after the session significantly went down compared with the ACTH value before the session (P<0.01: two-way ANOVA) .

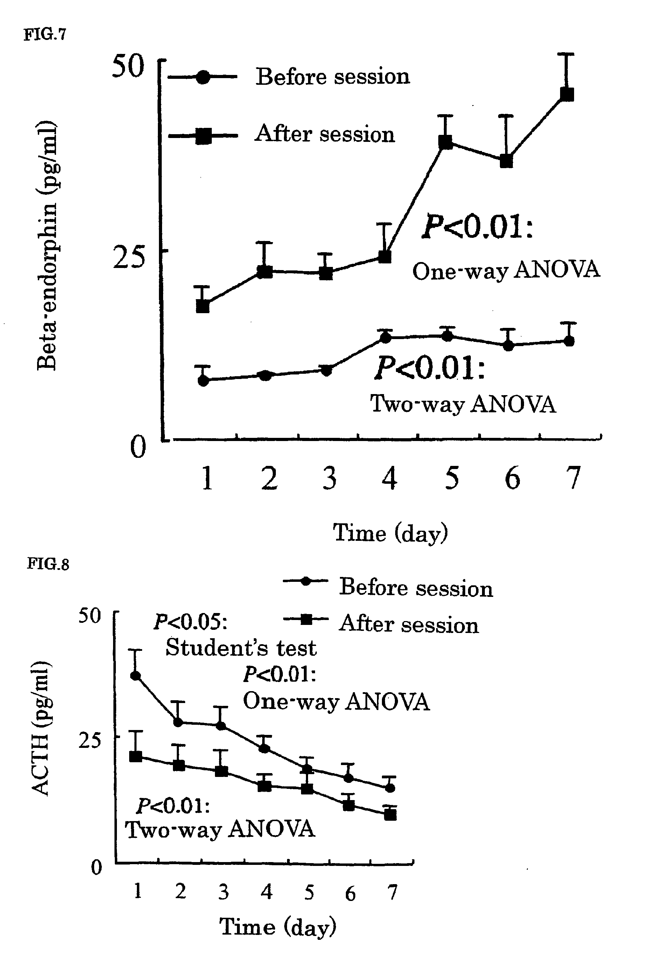

Fig.7 showed the beta-endorphin values and in all the sessions, the beta-endorphin values after the session constantly rose compared with beta-endorphin values before the sessions (P<0.01: two-way ANOVA). The ratio of the rise in each session was 2.29, 2.66, 2.44, 1.80, 2.89, 3.00, and 3.53 times, respectively. As a result of statistical analysis, compared with the gradual rise of the values after the session as the session progressed (P<0.01: one-way ANOVA), no statistical significance was observed in the changes of the values before the session for 7 days.

### Subject animals with spinal damage

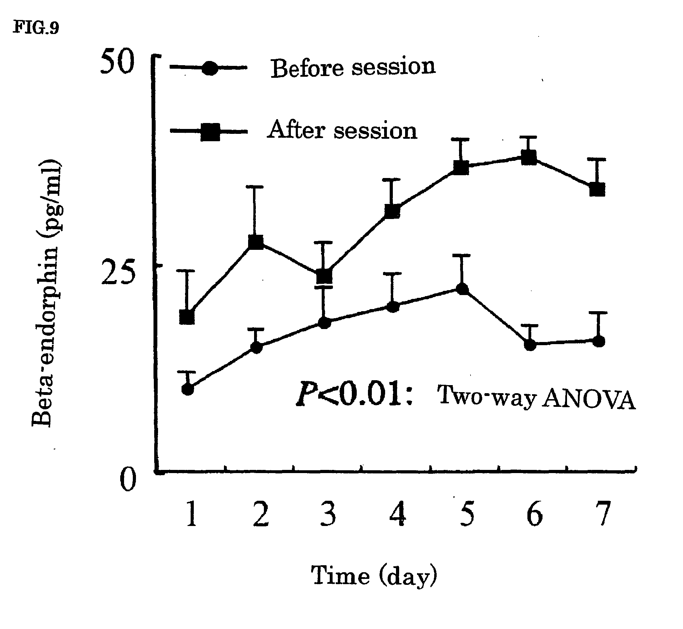

As shown in Fig.8, the ACTH values in the plasma before the 1st day of the session in subject animals with spinal damage (37.18±5.02pg/ml) were significantly high (P<0.05: student's test) compared with the ACTH values in normal subject animals (22.04±2.08pg/ml) or subject animals with tumor (21.06±4.43pg/ml), and during the experiments, the ACTH values gradually went down and as a result, the values went down to 15±2.17pg/ml (P<0.01:one-way ANOVA). The ACTH values before and after each session was significantly low compared with the values before the session (P<0.01:two-way ANOVA).

Further, as shown in Fig. 9, although clear characteristics in changes in each experiment of beta-endorphin were not suggested, the values after each session were higher than the values before the session (P<0.01:two-way ANOVA).

The heretofore mentioned results suggest that electric field exposure does not seriously affect stress hormone values (ACTH values and beta-endorphin values) of normal animals.

In the subject animals with spinal damage, the ACTH values after the session went down compared with the values before the session, which shows that the stress reaction related to pain sense has been tranquilized by electric fields. Further, the ACTH values before the session show remarkable degradation depending on the days. This at least shows that the stress caused by pain becomes tranquilized by the electric field treatment day by day.

Beta-endorphin values after the session in the subject animals suffering from pain caused by tumor or spinal damage dramatically rose. It is considered that the central pain-tranquilizing system of the subject animals has been up-regulated through the unknown pathways by the electric field.

Stress-related hormone values such as ACTH, beta-endorphin and the like of normal animals subject to extremely low electric field exposure are maintained without any influence. However, in the subject animals suffering from pain, the presence of the electric fields is considered to somewhat affect stress-related endocrine system. That is, it is well known that ACTH and beta-endorphin generated and secreted from proopiomelanocortin are treated at hypophysis and that ACTH is synthesized at anterior lobe and intermediate lobe, but is decomposed only at intermediate lobe. On the other hand, it is publicly known that although beta-endorphin is also synthesized in anterior lobe and intermediate lobe, the beta-endorphin is more quickly decomposed in intermediate lobe than in anterior lobe. Thus, it is considered that the synthetic pathways of beta-endorphin and the decomposition pathways of ACTH at hypophysis particularly at intermediate lobe are activated indirectly or non-indirectly.

### (Example 2)

### (Treating and improving apparatus)

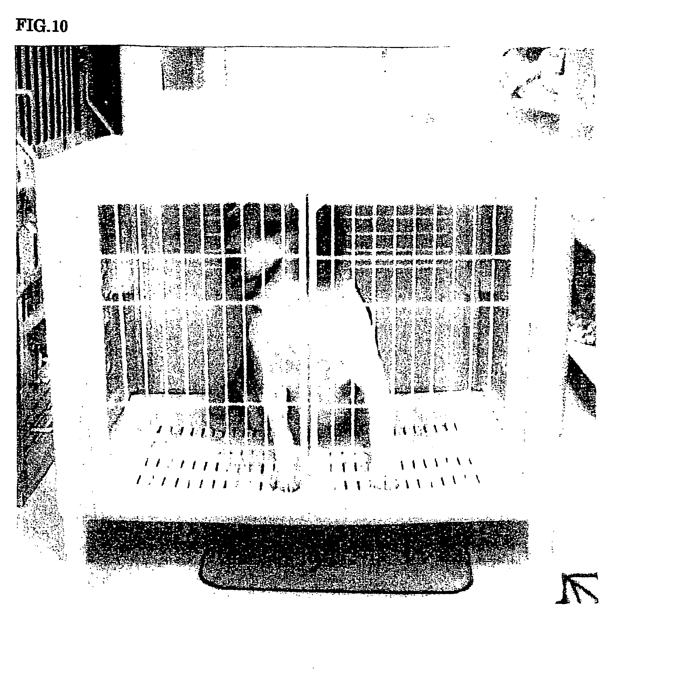

The same apparatus as in Example 1 was used as a treating and improving apparatus. Fig.10 shows the state in which a subject animal is kept in the cage.

### (Subject animal)

A subject animal is a dog that visited Nihon University animal hospital after diagnosed as chronic rheumatic arthritis by a veterinarian in general practice. The breed of the dog was a female Shetland sheep dog, 10 years and 1 month. The dog visited the hospital with the major complaint of ananastasia. The symptom spread from lameness in a hind limb to lameness in a forelimb. The case was suspected tobe early-phase rheumatic arthritis (eRA) and the later mentioned CRP value was measured. As a result, the CRP value showed high level of 2.4 mg/dl compared with a normal state.

### (Plasma parameter)

CRP (C-reactive protein) is one of acute-phase proteins which is not detected in normal blood and therefore, it is useful as an inflammatory marker forhumanbeings. This value indicates the presence of inflammatory disease belonging to these diseases such as rheumatic joint disease and the like and serves to judge disease progress.

It is said that positiveness of the CRP test goes abreast of intensity of disease symptom and in particular, in rheumatic disease with seesawing disease progression, exacerbation and lowering thereof can be judged by repeating examination. CRP positive is a diagnostic criterium of early-phase rheumatic arthritis in Japan College of Rheumatology and the relationship between the CRP positive and chronic rheumatic arthritis is suggested.

Considering that the CRP value can be used as judgment criteria of disease progression in dogs as well, the CRP value before and after the electric field exposure was measured. In a CRP measuring method, blood sample was obtained from the forelimb artery of a subject animal by conventional means, followed by separating blood plasma and using it for examination. A dog CRP concentration measuring apparatus (manufactured by Arrows Co., Ltd.) was used as a measuring apparatus and Dog CRP measuring reagent kit (manufactured by Arrows Co., Ltd.) was used as a kit.

### (Experimental results)

### Normal subject animals

In order to compare with the case of chronic rheumatic arthritis, 8 normal beagle dogs (4 male dogs and 4 female dogs) were used to measure the CRP values. As shown in table 1, the average value for males was 0.18 mg/dl, the average value for females was 0.45 mg/dl, the average value of females was relatively high, and the average for both was 0.31 mg/dl. The average value was substantially the same as the already reported result of CRP measurement.

**(Table 1)**

| CRP values of 8 normal dogs (mq/dl) | | | |
|---|---|---|---|
| Male (n=4) | | Female (n=4) | |
| No1 0.05 | | No5 | 0.3 |
| No2 | 0.1 | No6 | 1.0 |
| No3 | 0.55 | No7 | 0.3 |
| No4 | 0.0 | No8 | 0.2 |
| Average | 0.18 | Average | 0.45 |
| Total average (n=8) | | 0.31 | |

### Subject animal with chronic rheumatic arthritis

### CRP values

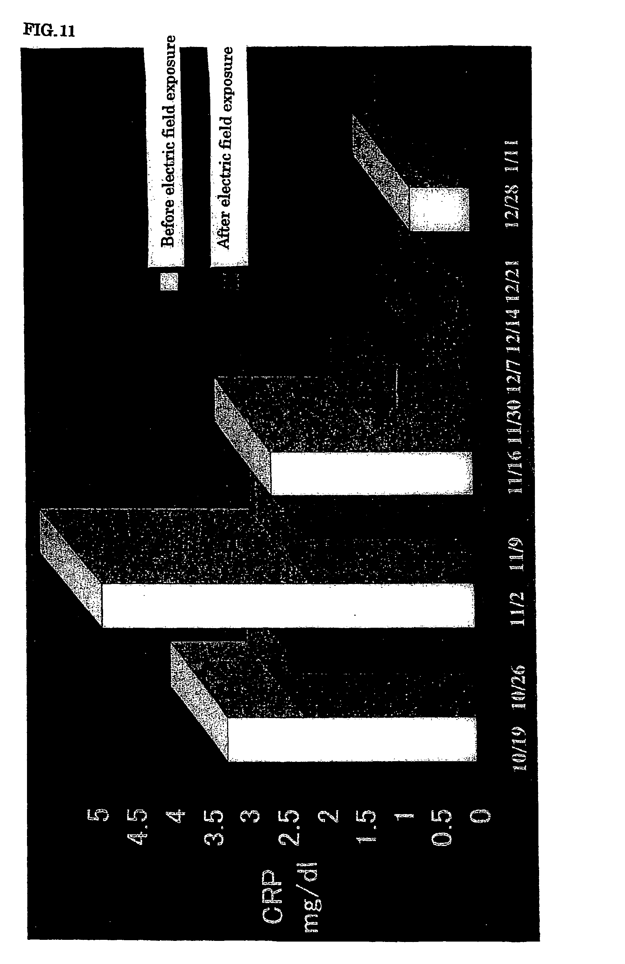

Subject animals with chronic rheumatic arthritis were treated by the electric field exposure. In the treatment, the electric field was exposed for two hours. The result is shown in Fig. 11 . CRP value measured before the electric field exposure when the subject animal visited the hospital was shown in white and the CRP value measured after the electric field exposure when the subject animal visited the hospital the following week was shown in gray. In the figure, the vertical axis shows the CRP measurement value and the horizontal axis shows the date.

It is observed that before and after the electric field exposure, there are changes in the CRP value. In addition, from November 16^{th}, the subject animal was treated with shorter intervals.

Next, since the CRP value decreased showing the effect of the electric field exposure, and since greater effect could be expected in serial electric field exposure everyday than the electric field exposure once a week, the subject animal was treated by the electric field exposure everyday.

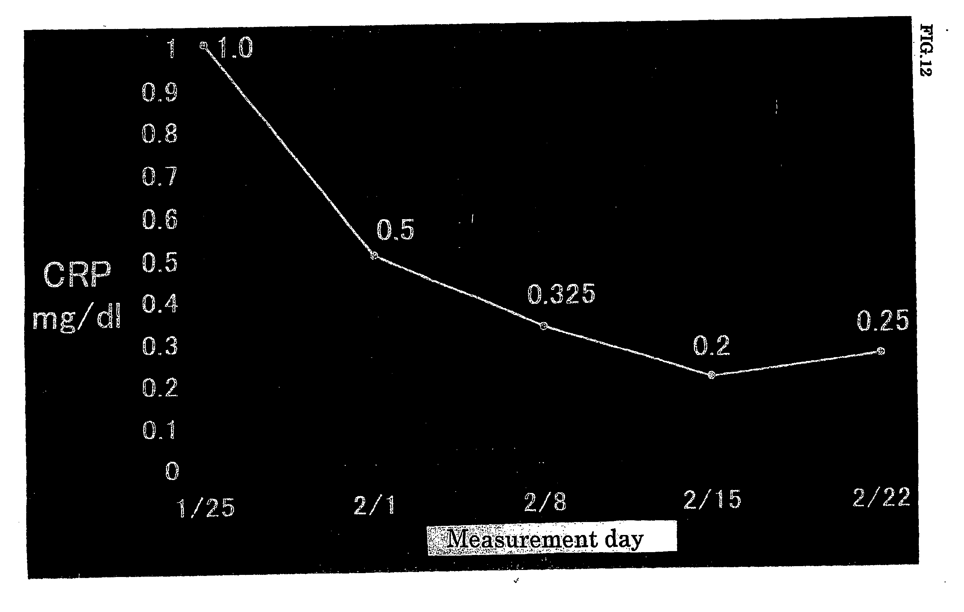

Fig. 12 shows the result of treatment by the electric field exposure everyday. From January 25^{th} to February 22^{nd} after the term of treatment by the electric field exposure a week, electric field was exposed to the subject animal for 2 hours everyday and the CRP value was measured every other week. It is observed that by conducting treatment by the electric field exposure continuously rather than conducting treatment by the electric field exposure a week, the CRP decreases and the greater effect can be obtained.

### Beta-endorphin value

Other than the CRP value, for the purpose of monitoring the state of the subject animal at the time of conducting electric field exposure, the beta-endorphin value in the blood was measured. Beta-endorphin is peptide hormone secreted from anterior lobe and intermediate lobe of hypophysis and is a substance with morphine-like tranquilizing effect. Beta-endorphin is said to be secreted when feeling comfortable or feeling happy and the average value for dogs is said to be 4 to 5 pg/ml.

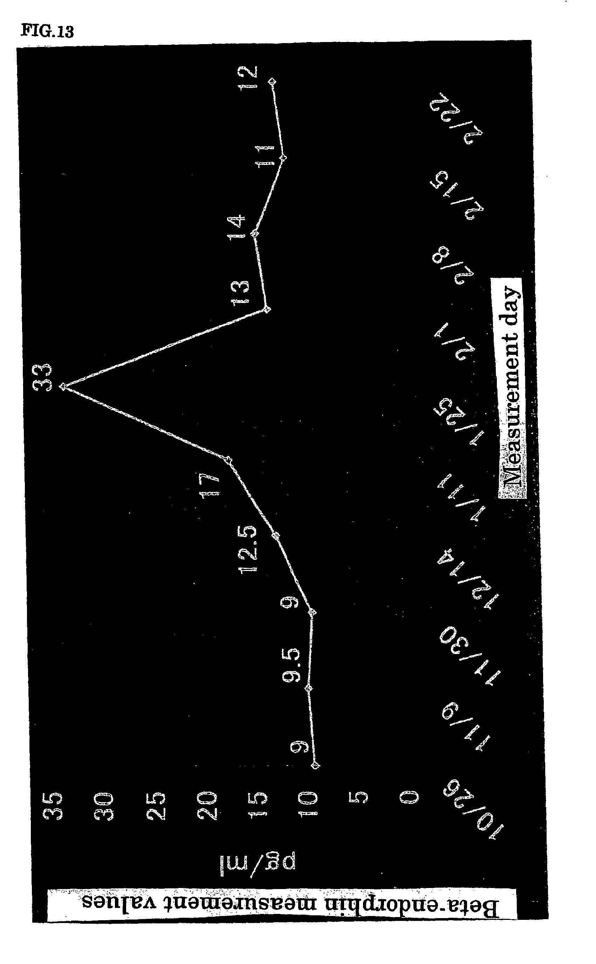

Fig.13 is a figure showing the beta-endorphin value of the subject animal. Compared with the average value for dogs, the value is observed to be high on the whole. In addition, on January 25^{th} at the time of starting electric field exposure continuously everyday, the great rise of beta-endorphin is temporarily observed.

For reference, when the beta-endorphin value of normal subject animal was measured, the value ranged from 8 to 15 pg/ml.

### MRI observation

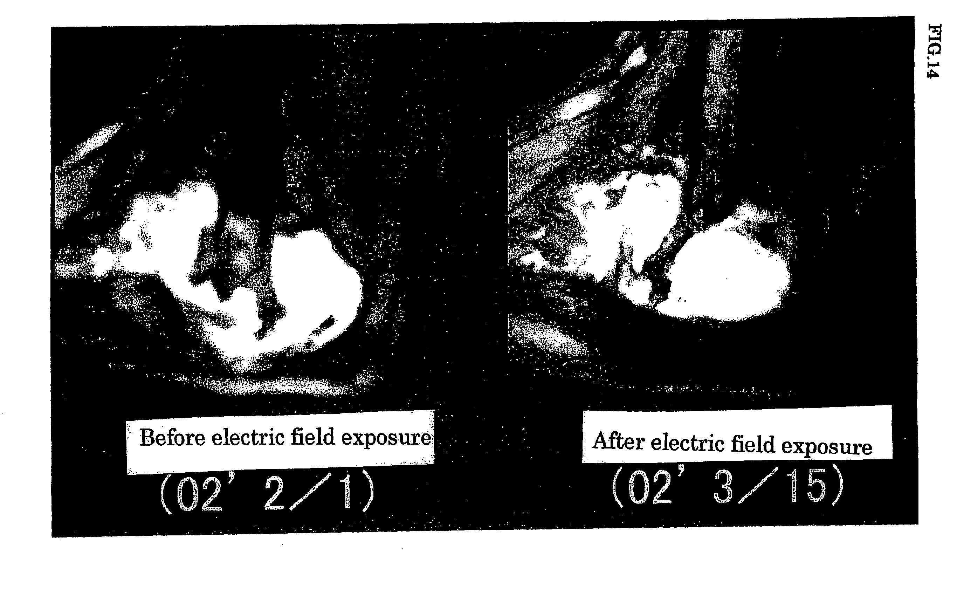

MRI imaging was conducted to a left elbow joint of the subject animal. When the T2 weighted images of MRI were compared before and after the electric field exposure, partial necrosis was acknowledged before the electric field exposure (Fig.14, left) and the necrosis part showed low signal, while active pannus showed high signal. On the other hand, after the electric field exposure (Fig.14, right), active pannus further disappeared and the high signal area remarkably decreased. This shows that organic improvement in rheumatism affected area was recognized by the electric field exposure.

As heretofore mentioned, summing up the changes before and after the treatment of subject animals suffering from chronic rheumatic arthritis, the CRP value that was higher than the normal value before the treatment lowered after the treatment. That is, it is said that by the lowering of the CRP value, the symptoms of rheumatism was improved. As shown in theMRI images in Fig. 14, since the area corresponding to the active pannus diminished, organic improvement was observed. In addition, after the treatment, since the uprise of beta-endorphin value was observed, it is considered that the electric field exposure has tranquilizing effect on pain caused by rheumatism.

### (Example 3)

### (Treating and improving apparatus)

The same apparatus as used in Examples 1 and 2 was used as a treating and improving apparatus.

### (Subject animals)

Subject animals are 12 dogs suffering from chronic rheumatic arthritis that visited the Nihon University animal hospital.

### (Experimental result)

### CRP value

Subject animals suffering from chronic rheumatic arthritis were treated by the electric field exposure. In the treatment, the electric field exposure was conducted for 2 hours a week. The CRP value was measured by the same method as in the Example 2 before and after the exposure. The CRP value before and after the exposure each week is shown in table 2. From the table, it is found that in any subject animal, the CRP value decreased by the electric field exposure and the remarkable effect can be obtained by repeating exposure.

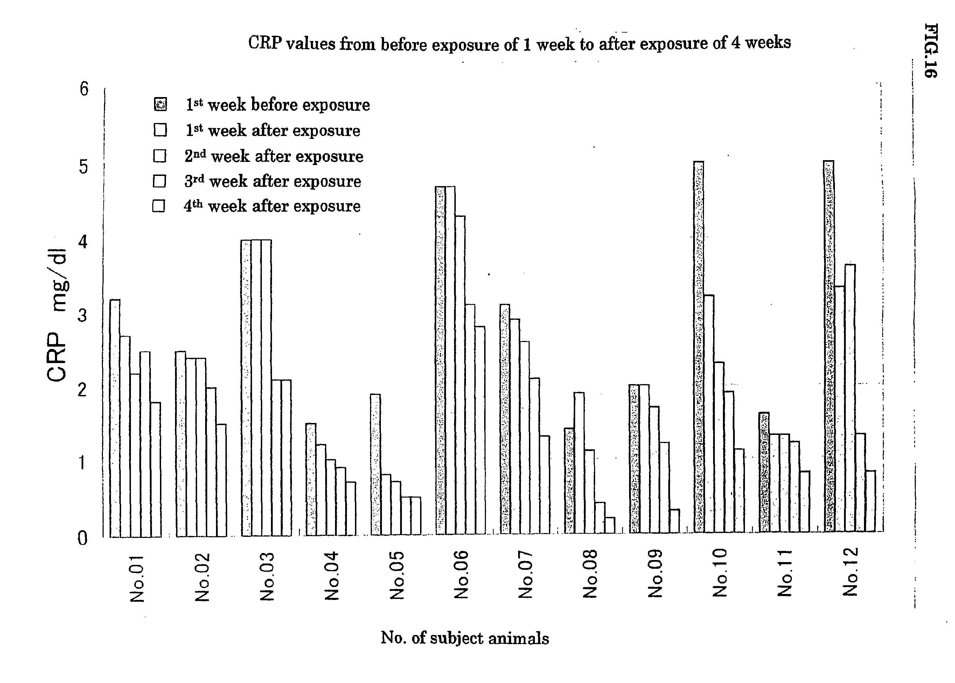

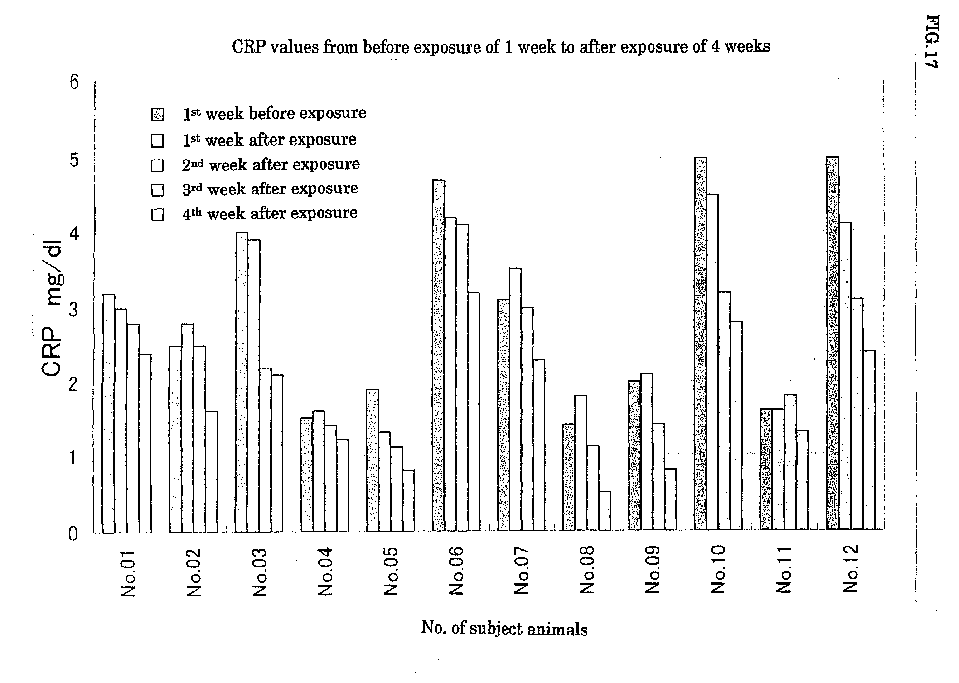

Figs. 16 and 17 show the CRP value of each subject animal after and before the electric field exposure. In the figures, dark gray shows the CRP 1 week before the exposure, namely, before the treatment. Pale gray shows the CRP value 1 week to 4 weeks after or before the exposure. According to this, although examples include some increase in the CRP, the CRP value more or less decreases. That is, although it was described that the shorter intervals for exposure are more effective, it shows that even when the intervals of exposure elongate, by the repeated electric field exposure, the CRP value decreases and the treatment effect is shown.

**(Table 2)**

| CRP values of subject animals before and after electric field exposure (1 to 4 weeks) unit : mg/dl | | | | | | | | | |
|---|---|---|---|---|---|---|---|---|---|
| Sex | No | 1 week | | 2 weeks | | 3 weeks | | 4 weeks | |
| | | Before exposure | After exposure | Before exposure | After exposure | Before exposure | After exposure | Before exposure | After exposure |
| Female | No.01 | 3.2 | 2.7 | 3 | 2.2 | 2.8 | 2.5 | 2.4 | 1.8 |
| Female | No.02 | 2.5 | 2.4 | 2.8 | 2.4 | 2.5 | 2 | 1.6 | 1.5 |
| Female | No.03 | 4 | 4 | 3.9 | 4 | 2.2 | 2.1 | 2.1 | 2.1 |
| Male | No.04 | 1.5 | 1.2 | 1.6 | 1 | 1.4 | 0.9 | 1.2 | 0.7 |
| Male | No.05 | 1.9 | 0.8 | 1.3 | 0.7 | 1.1 | 0.5 | 0.8 | 0.5 |
| Female | No.06 | 4.7 | 4.7 | 4.2 | 4.3 | 4.1 | 3.1 | 3.2 | 2.8 |
| Male | No.07 | 3.1 | 2.9 | 3.5 | 2.6 | 3 | 2.1 | 2.3 | 1.3 |
| Female | No.08 | 1.4 | 1.9 | 1.8 | 1.1 | 1.1 | 0.4 | 0.5 | 0.2 |
| Male | No.09 | 2 | 2 | 2.1 | 1.7 | 1.4 | 1.2 | 0.8 | 0.3 |
| Female | No.10 | 5 | 3.2 | 4.5 | 2.3 | 3.2 | 1.9 | 2.8 | 1.1 |
| Male | No.11 | 1.6 | 1.3 | 1.6 | 1.3 | 1.8 | 1.2 | 1.3 | 0.8 |
| Male | No.12 | 5 | 3.3 | 4.1 | 3.6 | 3.1 | 1.3 | 2.4 | 0.8 |

Although the explanation was made with regard to the Examples to dogs, by preparing a pair of electrodes with appropriate size and by arranging them with appropriate intervals, other mammalian animals such as cats and the like and human beings and the like can be treated and improved.

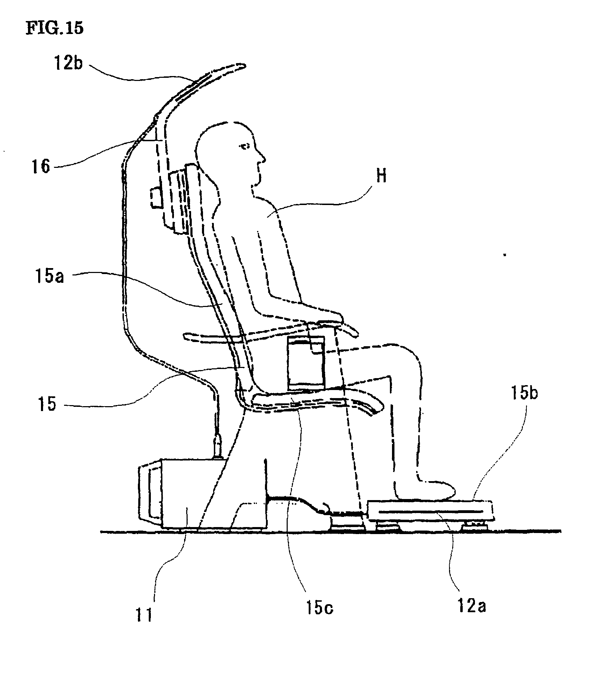

When the mammalian animals are human beings, as shown in Fig.15, the apparatus can be prepared as the one which exposes electric field to a human being H sitting on a chair 15 (placing table) provided with a back rest portion 15 a, a foot rest portion 15 b, and a seat plate portion 15 c. Inside of a foot rest portion 15 b, one of a pair of electrodes 12 a is arranged. Another of said pair of electrodes 12 b is arranged in an electrode supporting portion 16 which extends from the back rest portion 15 a. In other words, since there is an air gap between a head portion of a human being H and an electrode 12 b, they do not contact with each other, and since the electrode 12 a is inside of a foot rest portion 15 b, it does not contact with a foot portion of the human being H, either. Therefore, a pair of electrodes 12 is arranged in a non-contact state to a human being H.

By applying voltage from a high voltage generating unit 11 to a pair of electrodes 12a and 12b, electric field is generated between a pair of electrodes 12. By the disposition of electrodes 12, electric field can be exposed not only to affected areas of rheumatism or those of pain, but also to a foot portion from a head portion of a human H. In addition, a high voltage generating unit 11 includes a controller (not illustrated) and the controller can control frequency, applied voltage, exposure time, and the like. Depending on height or weight of a human being H, these values can be controlled.

### Industrial applicability

According to the method and the apparatus of the present invention, rheumatism of mammalian animals including pet animals such as dogs, cats, and the like and human beings can be treated or improved by a non-pharmacological method. In addition, pain caused by rheumatism, tumorpinal damage and the like can also be treated or improved by the non-pharmacological method.

## Claims

1. Non-pharmacological methods of treating and improving rheumatism of mammalian animals that treat and improve rheumatism of mammalian animals non-pharmacologically, wherein electrodes are placed in a non-contact state with mammalian animals and the electric field is exposed to a whole or a part including rheumatism affected areas of said mammalian animals.

2. The non-pharmacological method of treating and improving rheumatism of mammalian animals as set forth in claim 1, wherein said electric field is extremely low frequency AC electric field.

3. The non-pharmacological method of treating and improving rheumatism of mammalian animals as set forth in claim 2, wherein said electric field is further overlapped with DC electric field.

4. The non-pharmacological method of treating and improving rheumatism of mammalian animals as set forth in claim 1, wherein said electric field is exposed to for a certain period of time in a day, repeating this more than once a week.

5. The non-pharmacological method of treating and improving rheumatism of mammalian animals as set forth in claim 1, wherein said mammalian animals are dogs, cats, or human beings.

6. An apparatus for treating or improving rheumatism of mammalian animals, provided with a pair of electrodes that is capable of exposing the electric field to a whole or a part that includes rheumatism affected areas of said mammalian animals and a power source that applies voltage to said pair of electrodes.

7. The apparatus for treating or improving rheumatism of mammalian animals as set forth in claim 6, wherein said electric field is the extremely low frequency AC electric field.

8. The apparatus for treating or improving rheumatism as set forth in claim 7, wherein said electric field is further overlapped with DC electric field.

9. The apparatus for treating or improving rheumatism as set forth in claim 6, wherein at least one of applied voltage, frequency, and exposure time is further provided with a controllable electric power source controller.

10. The apparatus for treating or improving rheumatism as set forth in claim 6, wherein said mammalian animals are dogs, cats, or human beings.

11. Non-pharmacological methods of treating and improving pain caused by rheumatism, tumor, and spinal damage of mammalian animals that treat and improve pain of mammalian animals caused by rheumatism, tumor, and spinal damage non-pharmacologically, wherein electrodes are placed in a non-contact state with mammalian animals and the electric field is exposed to a whole or a part including rheumatism affected areas of said mammalian animals.

12. The non-pharmacological method of treating and improving pain of mammalian animals as set forth in claim 11, wherein said electric field is extremely low frequency AC electric field.

13. The non-pharmacological method of treating and improving pain of mammalian animals as set forth in claim 12, wherein said electric field is further overlapped with DC electric field.

14. The non-pharmacological method of treating and improving pain of mammalian animals as set forth in claim 11, wherein said electric field is exposed to for a certain period of time in a day, repeating this more than once a week.

15. The non-pharmacological method of treating and improving pain of mammalian animals as set forth in claim 11, wherein said mammalian animals are dogs, cats, or human beings.

16. An apparatus for treating or improving pain of mammalian animals caused by rheumatism, tumor, and spinal damage, provided with apair of electrodes that is capable of exposing the electric field to a whole or a part that includes rheumatism affected areas of said mammalian animals and a power source that applies voltage to said pair of electrodes.

17. The apparatus for treating or improving pain of mammalian animals as set forth in claim 16, wherein said electric field is the extremely low frequency AC electric field.

18. The apparatus for treating or improving pain of mammalian animals as set forth in claim 17, wherein said electric field is further overlapped with DC electric field.

19. The apparatus for treating or improving pain as set forth in claim 16, wherein at least one of applied voltage, frequency, and exposure time is further provided with a controllable electric power source controller.

20. The apparatus for treating or improving pain as set forth in claim 16, wherein said mammalian animals are dogs, cats, or human beings.
